# EUROPEAN PATENT SPECIFICATION

(11) **EP 2 578 544 B1**
(45) Date of publication and mention of the grant of the patent: **12.12.2018**
(21) Application number: 11184431.2
(22) Date of filing: 07.10.2011
(51) Int. Cl.: C02F 1/74, C02F 101/10, C02F 103/18, C02F 1/72, C02F 3/34, C02F 1/68

(54) **Method and system for controlling treatment of effluent from seawater flue gas scrubber**
Verfahren und System zum Steuern der Behandlung des Abwassers aus einem Meerwasser-Rauchgaswäscher
Procédé et système pour contrôler le traitement des effluents issus d'un épurateur de gaz de fumée à l'aide d'eau de mer

(43) Date of publication of application: 10.04.2013
(73) Proprietor: General Electric Technology GmbH, 5400 Baden (CH)
(72) Inventor: Brogaard, Fredrik Jens, 352 36 Växjö (SE); Larsson, Mikael, 431 44 Mölndal (SE)
(74) Representative: General Electric Technology GmbH

(56) References cited:
- EP-A2- 1 040 864
- WO-A1-01/41902
- WO-A1-88/07023
- GB-A- 2 159 508
- JP-A- 59 213 495
- DATABASE WPI Week 200940 Thomson Scientific, London, GB; AN 2009-K06413 XP002671082, "Load adjustable aeration device for recovering sea water after de-sulfurizing", -& CN 201 240 931 Y (YANG D) 20 May 2009 (2009-05-20)

## Description

### Field of the Invention

The present invention relates to a method of controlling the treatment of effluent seawater generated in the removal of sulfur dioxide from a process gas by contacting the process gas containing sulfur dioxide with seawater.

The present invention further relates to a seawater oxidation basin system for treating effluent seawater generated in a wet scrubber in which a process gas is brought into contact with seawater for removal of sulfur dioxide from said process gas.

### Background of the Invention

Process gases containing sulfur dioxide, SO₂, are generated in many industrial processes. One such industrial process is the combustion of a fuel, such as coal, oil, peat, waste, etc., in a combustion plant, such as a power plant. In such a power plant, a hot process gas, often referred to as a flue gas, is generated containing pollutants including acid gases, such as sulfur dioxide, SO₂. It is necessary to remove as much of the acid gases as possible from the flue gas before the flue gas may be emitted to the ambient air. Another example of an industrial process in which a process gas containing pollutants is generated is the electrolytic production of aluminum from alumina. In that process, a process gas containing sulfur dioxide, SO₂, is generated within venting hoods of the electrolytic cells.

WO 2008/105212 discloses a boiler system comprising a boiler, a steam turbine system, and a seawater scrubber. The boiler generates, by combustion of a fuel, high-pressure steam utilized in the steam turbine system for generating electric power. Seawater is collected from the ocean, and is utilized as a cooling medium in a condenser of the steam turbine system. The seawater is then utilized in the seawater scrubber for absorbing sulfur dioxide, SO₂, from flue gas generated in the boiler. Sulfur dioxide, SO₂, is absorbed in the seawater and forms sulfite and/or bisulfite ions. Effluent seawater from the seawater scrubber is forwarded to an aeration pond. Air is bubbled through the effluent seawater in the aeration pond for oxidation by means of oxygen gas contained in the air, of the sulfite and/or bisulfite ions to sulfate ions for release back to the ocean together with the effluent seawater.

CN 201240931 discloses a load adjustable aeration device for recovering sea water after desulfurization. Aeration pipes extend transversely across an aeration tank and are provided with respective valves.

JP 59 213495 discloses a process for treating a condensate generated in a reverse osmosis membrane separator for a desalination plant. The condensate is aerated in three separate tanks with individual control of pH by alkali supply.

### Summary of the Invention

An object of the present invention is to provide a method of controlling the treatment of effluent seawater generated in the removal of sulfur dioxide from a process gas according to claim 1.

An advantage of this method is that the oxidation enhancing substance can be supplied where it is needed the most, thus obtaining efficient oxidation of sulfite and/or bisulfite with minimum consumption of oxidation enhancing substance and, hence, minimum operating costs.

According to one embodiment the second supply position is located downstream with respect to the direction of effluent seawater flow along the oxidation basin, of the first supply position. An advantage of this embodiment is that the progress of the oxidation process along the oxidation basin can be controlled to make the oxidation process as efficient as possible.

According to one embodiment, the oxidation enhancing substance further comprises at least one of: an oxidation enhancing catalyst, and an oxidation enhancing enzyme. An advantage of this embodiment is that the supply of one or several different oxidation enhancing substances to provide the most efficient control may be selected for control.

According to one embodiment, the method further comprises measuring at least one parameter relating to the oxidation of sulfite and/or bisulfite selected from a group of parameters comprising: sulfite concentration, oxygen concentration, and pH. An advantage of this embodiment is that sulfite concentration, oxygen concentration, and pH are all related to the oxidation process in the oxidation basin system, and provides relevant information about the progress of the oxidation process. Furthermore, sulfite concentration, oxygen concentration, and pH are often required measurements per regulatory requirements concerning the return of the effluent seawater to the ocean.

According to one embodiment, the method further comprises independently controlling a first amount of an oxidation enhancing substance supplied in a first supply position and independently controlling a second amount of an oxidation enhancing substance supplied in a second supply position. An advantage of this embodiment is that controlling the first and the second amounts of oxidation enhancing substance(s) independently provides for improved control of the oxidation process.

According to one embodiment, the method further comprises supplying one or more oxidation enhancing substances which may be the same or differ in at least first, second, third, and fourth supply positions arranged consecutively along the oxidation basin, and measuring at least one parameter in at least first, second, third and fourth measurement positions arranged consecutively along the oxidation basin and downstream of the respective supply positions. An advantage of this embodiment is that the control of the oxidation process in the oxidation basin is further improved, resulting in lower operating costs and reduced risk of violating regulatory requirements.

According to one embodiment, the method further comprises measuring a concentration of sulfite and/or bisulfite in a first measurement position, and controlling a first amount of oxidation enhancing substance supplied in a first supply position based on the measured concentration of sulfite and/or bisulfite. An advantage of this embodiment is that measuring the sulfite and/or bisulfite concentration and controlling the amount of oxidation enhancing substance supplied upstream of such measurement provides for a fast and accurate response to variations in the oxidation requirements.

A further object of the present invention is to provide a seawater oxidation basin system for treating effluent seawater generated in a wet scrubber according to claim 8.

An advantage of this oxidation basin system is that it is efficient with regard to investment, operation and maintenance costs, since the size, capacity and energy consumption of the oxidation enhancing substance supply devices and the oxidation basin can be reduced, due to a more efficient utilization of the one or more oxidation enhancing substances supplied to the oxidation basin system.

The oxidation basin system further comprises a control unit for controlling, based on a parameter measured by the first and second water quality sensors, a first amount of oxidation enhancing substance which is an oxygen containing gas supplied by one of the first and second supply pipes independently from a second amount of oxidation enhancing substance which is an oxygen containing gas supplied by the other one of the first and second supply pipes. An advantage of this embodiment is that it provides for an automatic and fast response to variations in the oxidation process.

According to one embodiment, the first and second supply pipes are provided with individual control valves for controlling an amount of oxidation enhancing substance supplied by each respective supply pipe. An advantage of this embodiment is that each of the first and the second supply pipes may be controlled individually.

According to one embodiment, the first and second supply pipes are connected to individual blowing devices for controlling an amount of oxygen containing gas supplied by each respective supply pipe. Controlling the blowers provides for direct control of energy consumed by each supply pipe.

According to one embodiment, the oxidation basin system comprises 3 to 10 consecutive supply pipes. According to a further embodiment the oxidation basin system comprises 3 to 10 consecutive water quality sensors. Utilizing fewer than 3 consecutive water quality sensors, and/or fewer than 3 consecutive supply pipes reduces control over the oxidation process, hence increasing energy consumption and increasing the risk of exceeding regulatory requirements. Utilizing more than 10 consecutive water quality sensors and/or more than 10 consecutive supply pipes increases investment and maintenance costs, without substantially improving control over the oxidation process.

According to a further aspect, there is provided a seawater based process gas cleaning system according to claim 15 comprising:
a wet scrubber for process gas contact with seawater for removal of sulfur dioxide from said process gas, and
an oxidation basin system for treating effluent seawater generated in the wet scrubber and for removing sulfur dioxide from the process gas.

Further objects and features of the present invention will be apparent from the description and the claims.

### Brief description of the Drawings

The invention will now be described in more detail with reference to the appended drawings in which:
Fig. 1 is a schematic side cross-section view of a power plant with a seawater based gas cleaning system.
Fig. 2 is a schematic side cross-section view illustrating an oxidation basin system in accordance with a first embodiment.
Fig. 3 is a schematic side cross-section view illustrating an oxidation basin system in accordance with a second embodiment.
Fig. 4 is a diagram illustrating measured profiles of oxygen, sulfite and pH along an oxidation basin.
Fig. 5a is a diagram illustrating an example in which the concentration of sulfite is too high.
Fig. 5b is a diagram illustrating an example in which the concentration of sulfite is reduced too fast.
Fig. 6a is a diagram illustrating an example in which the concentration of oxygen is too low.
Fig. 6b is a diagram illustrating an example in which the concentration of oxygen increases too fast.
Fig. 7a is a diagram illustrating an example in which the pH value is too low.
Fig. 7b is a diagram illustrating an example in which the pH value increases too fast.
Fig. 8 is a schematic top view illustrating an oxidation basin system in accordance with a third embodiment.
Fig. 9 is a perspective view of a sulphite sensor.
Fig. 10 is a schematic cross-sectional side view of a sulphite sensor.
Fig. 11 is a flow chart of a method of measuring sulphite.
Fig. 12a is a graph of voltage level over time from a method of measuring sulphite.
Fig. 12b is a simulation plot of voltage level pulses from a method of measuring sulphite.
Fig. 12c is a simulation plot of a voltage corresponding to a current response generated by voltage pulses in Fig. 12b.

### Description of preferred Embodiments

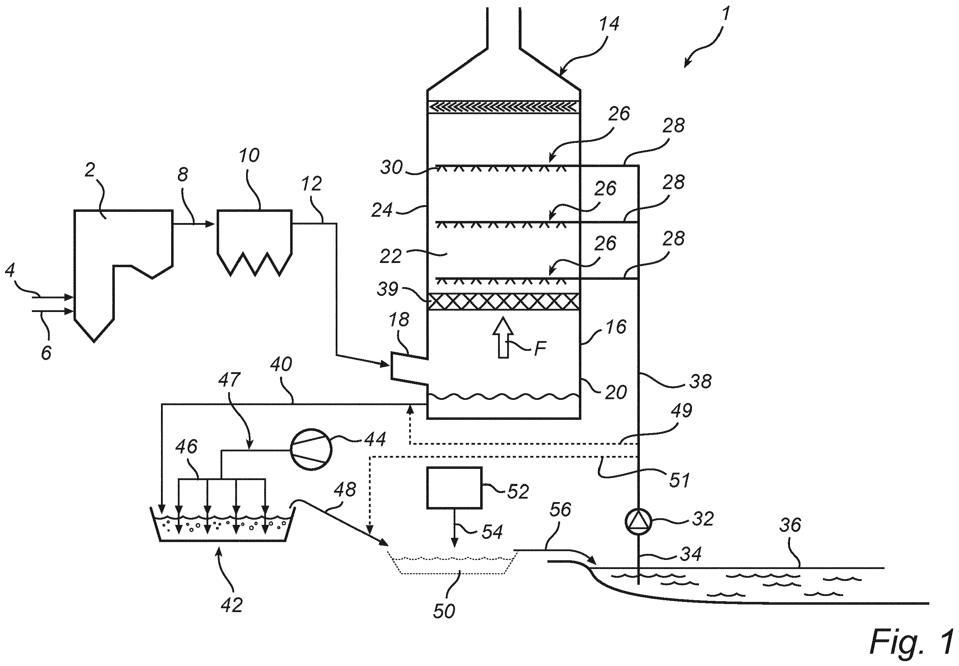

Fig. 1 is a schematic side cross-section view illustrating a power plant 1. Power plant 1 comprises a boiler 2 in which a fuel, such as coal, oil, peat, natural gas, or waste, supplied via feeding pipe 4 is combusted in the presence of oxygen, supplied via oxygen supply duct 6. Oxygen may, for example, be supplied in the form of air and/or in the form of a mixture of oxygen gas and recirculated gases, in case boiler 2 is a so-called "oxy-fuel" boiler. The combustion of fuel generates a hot process gas in the form of a flue gas. Sulfur species contained in the fuel upon combustion form, at least partly, sulfur dioxide, SO₂, which forms part of the flue gas.

The flue gas may flow from boiler 2 via a fluidly connected duct 8, to an optional dust removal device in the form of an electrostatic precipitator 10. The electrostatic precipitator 10, an example of which is described in US 4,502,872, serves to remove dust particles from the flue gas. As an alternative, another type of dust removal device may be used, such as for example, a fabric filter as described in US 4,336,035.

Flue gas, from which most of the dust particles have been removed, flows from the electrostatic precipitator 10 via a fluidly connected duct 12 to a seawater scrubber 14. Seawater scrubber 14 comprises a wet scrubber tower 16. An inlet 18 is arranged at a lower portion 20 of wet scrubber tower 16. Duct 12 is fluidly connected to inlet 18 such that flue gas flowing from electrostatic precipitator 10 via duct 12 may enter interior 22 of wet scrubber tower 16 via inlet 18.

After entering interior 22, flue gas flows vertically upward through wet scrubber tower 16, as indicated by arrow F. Central portion 24 of wet scrubber tower 16 is equipped with a number of spray arrangements 26 arranged vertically one above each other. In the example of Fig. 1, there are three such spray arrangements 26, and typically there are 1 to 20 such spray arrangements 26 in a wet scrubber tower 16. Each spray arrangement 26 comprises a supply pipe 28 and a number of nozzles 30 fluidly connected to each supply pipe 28. Seawater supplied via supply pipes 28 to nozzles 30 is atomized by means of nozzles 30 and contacts in interior 22 of wet scrubber tower 16, the flue gas for absorption of sulfur dioxide, SO₂, therefrom.

A pump 32 is arranged for pumping seawater via fluidly connected suction pipe 34 from ocean 36, and forwarding the seawater via fluidly connected pressure pipe 38 to fluidly connected supply pipes 28.

In accordance with an alternative embodiment, seawater supplied by pump 32 to pipes 28 may be seawater previously utilized as cooling water in steam turbine systems associated with boiler 2 prior to such seawater being utilized as scrubbing water in seawater scrubber 14.

Seawater atomized by means of nozzles 30 in interior 22 of wet scrubber tower 16 flows downwardly within wet scrubber tower 16 and absorbs sulfur dioxide from flue gas F flowing vertically upwardly within interior 22 of wet scrubber tower 16. As a result of such absorption of sulfur dioxide by the seawater, the seawater gradually turns into effluent seawater as it flows downwardly within interior 22 of wet scrubber tower 16. Effluent seawater is collected in lower portion 20 of wet scrubber tower 16 and is forwarded, via fluidly connected effluent pipe 40, from wet scrubber tower 16 to an oxidation basin system 42.

In accordance with an alternative embodiment, the seawater scrubber 14 may comprise one or more layers of a packing material 39 arranged within interior 22 of wet scrubber tower 16. Packing material 39 may be made from plastic, steel, wood, or another suitable material for enhanced gas-liquid contact. With packing material 39, nozzles 30 merely distribute seawater over packing material 39, rather than atomizing the seawater. Examples of packing material 39 include Mellapak™ (available from Sulzer Chemtech AG, Winterthur, CH) and Pall™ rings (available from Raschig GmbH, Ludwigshafen, DE).

Optionally, fresh seawater may be added to effluent seawater prior to further treatment of the effluent seawater. To this end, a pipe 49 may be fluidly connected to pressure pipe 38 to provide a flow of fresh seawater to fluidly connected effluent pipe 40 for effluent seawater flow to oxidation basin system 42. Hence, an intermixing of fresh seawater and effluent seawater occurs in pipe 40. As alternative, fresh seawater from pipe 49 may flow directly to oxidation basin system 42 for mixture with the effluent seawater therein. As a still further option, residual waters and/or condensates generated in boiler 2 or steam turbine systems associated therewith could be mixed with the effluent seawater.

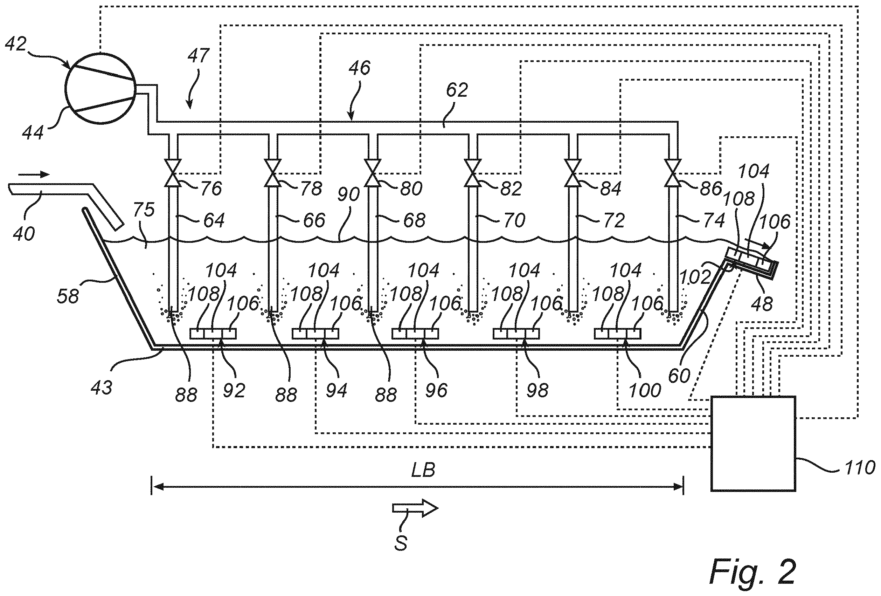

Oxidation basin system 42 comprises a blowing device in the form of a compressor or a blower 44 arranged for blowing, via fluidly connected ductwork 46, one or more oxidation enhancing substances in the form of an oxygen containing gas, such as air, into the effluent seawater. Blower 44 and ductwork 46 together form an oxygen supply system 47 for supplying oxygen to the effluent seawater. A more detailed description of the oxidation basin system 42 is provided below with reference to Fig. 2.

Optionally, effluent seawater may flow via a fluidly connected overflow pipe 48 from oxidation basin system 42 to an alkalization basin 50. Storage 52 of alkali agent is, optionally, arranged for supplying alkali agent via fluidly connected pipe 54 to basin 50. The alkali agent may, for example, be limestone or fresh seawater from the ocean, which serves to increase the pH of the effluent seawater if needed.

Effluent seawater finally flows via a fluidly connected overflow pipe 56 from alkalization basin 50 back to the ocean 36.

In accordance with an alternative embodiment, effluent seawater flows via overflow pipe 48 to the ocean 36 without passing any alkalization basin. In accordance with a further alternative embodiment, effluent seawater is mixed with fresh seawater prior to being discharged into the ocean 36. To this end, a pipe 51 may be fluidly connected to pressure pipe 38 for a flow of fresh seawater to fluidly connected overflow pipe 48. Hence, an intermixing of fresh seawater and effluent seawater occurs in pipe 48.

Fig. 2 illustrates the oxidation basin system 42 in more detail. Effluent seawater is supplied to an oxidation basin 43 of oxidation basin system 42 via fluidly connected pipe 40 at a first end 58, or "inlet end", of oxidation basin 43. Effluent seawater flows generally horizontally as indicated by arrow S along length LB of oxidation basin 43, from first end 58 to a second end 60, or "outlet end", of oxidation basin 43. At second end 60, effluent seawater overflows into fluidly connected overflow pipe 48 and leaves basin 43.

Oxidation basin system 42 further includes oxygen supply system 47 with ductwork 46. Ductwork 46 comprises a central distribution duct 62 extending horizontally along basin 43 from a point adjacent to first end 58 to a point adjacent to second end 60. Ductwork 46 further comprises supply pipes in the form of first, second, third, fourth, fifth, and sixth consecutive air distribution pipes 64, 66, 68, 70, 72, 74, respectively, fluidly connected to central distribution duct 62 and extending into effluent seawater 75 flowing horizontally through basin 43. The six air distribution pipes 64, 66, 68, 70, 72, 74 are arranged consecutively along length LB of basin 43, with first air distribution pipe 64 located in closest proximity to first end 58, second air distribution pipe 66 located downstream of the first pipe 64, etc., with the sixth air distribution pipe 74 located in closest proximity to second end 60. Each air distribution pipe 64, 66, 68, 70, 72, 74 is provided with a control device in the form of a control valve 76, 78, 80, 82, 84, 86 useful to control the flow of oxygen containing gas, such as air, through each respective air distribution pipe 64, 66, 68, 70, 72, 74. Blower 44 blows air into central distribution duct 62 and further into air distribution pipes 64, 66, 68, 70, 72, 74. The lower ends 88 of air distribution pipes 64, 66, 68, 70, 72, 74 are open and are arranged below liquid surface 90 of effluent seawater 75 in oxidation basin 43. Air that is blown by blower 44 flows via central distribution duct 62 and air distribution pipes 64, 66, 68, 70, 72, 74 to open lower ends 88. At open ends 88 air is dispersed and mixed with the effluent seawater. At least a portion of the oxygen content of the air thus dispersed and mixed with effluent seawater is dissolved in the effluent seawater and reacts to oxidize sulfite and/or bisulfate ions in accordance with the chemical reactions described in more detail below.

In accordance with an alternative embodiment, the oxygen supply system 47 may be operative for blowing an oxygen rich gas comprising more than 21% by volume oxygen, for example comprising 75-100 % by volume oxygen, into the effluent seawater of oxidation basin 43.

Oxidation basin system 42 may further comprise first, second, third, fourth, and fifth consecutive water quality sensors 92, 94, 96, 98, 100, immersed in effluent seawater 75 flowing through basin 43. The five water quality sensors 92, 94, 96, 98, 100 are arranged consecutively along length LB of basin 43, with the first water quality sensor 92 located in closest proximity to first end 58, the second water quality sensor 94 located downstream of the first sensor 92, etc., with the fifth sensor located in closest proximity to second end 60. A sixth, and last, water quality sensor 102 is arranged in overflow pipe 48. Each water quality sensor 92, 94, 96, 98, 100, 102 may comprise one or more detecting elements. In the embodiment illustrated in Fig. 2, each water quality sensor comprises a sulfite detecting element 104, an oxygen detecting element 106, and a pH detecting element 108.

Each water quality sensor 92, 94, 96, 98, 100, 102 is positioned to detect one or more parameters of the effluent seawater, as measured in that specific area where the water quality sensor in question is placed, and for sending a signal to a control unit 110. The control unit 110, which may be a process control computer, analyses the signals received from the respective water quality sensors 92, 94, 96, 98, 100, 102, and automatically controls, in accordance with principles described in more detail hereinafter, the setting of the respective control valves 76, 78, 80, 82, 84, 86 such that a suitable flow of oxygen containing gas is supplied to the effluent seawater via each of the air distribution pipes 64, 66, 68, 70, 72, 74. The control unit 110 may also automatically control the output of blower 44, such that a suitable amount of air is supplied to the ductwork 46 and further to the air distribution pipes 64, 66, 68, 70, 72, 74.

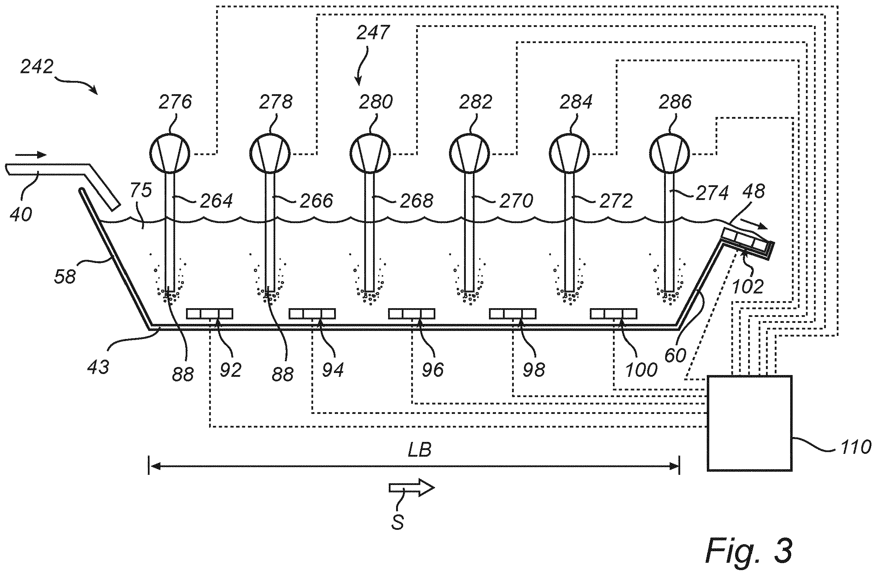

Fig. 3 is a schematic representation of an alternative oxidation basin system 242. Those features of the oxidation basin system 242 that are similar to features of oxidation basin system 42 share the same reference numerals. Effluent seawater 75 is supplied to oxidation basin 43 of the oxidation basin system 242 via fluidly connected pipe 40 at first end 58, being an inlet end, of oxidation basin 43. The effluent seawater flows, generally horizontally as indicated by arrow S, along the length LB of the oxidation basin 43, from the first end 58 to a second end 60, and leaves basin 43 via fluidly connected overflow pipe 48.

Oxidation basin system 242 further includes an oxygen supply system 247. The oxygen supply system 247 comprises first, second, third, fourth, fifth, and sixth air distribution pipes 264, 266, 268, 270, 272, and 274, each of which is fluidly connected to a control device in the form of an individual blower 276, 278, 280, 282, 284, 286, and extends into the effluent seawater 75 flowing horizontally through basin 43. The six air distribution pipes 264, 266, 268, 270, 272, 274 are intermittently spaced along the length LB of the basin 43. Air blown by blowers 276, 278, 280, 282, 284, 286 is dispersed and mixed with the effluent seawater 75 at open lower ends 88 of air distribution pipes 264, 266, 268, 270, 272, 274.

Oxidation basin system 242 may further comprise first, second, third, fourth, fifth and sixth water quality sensors, 92, 94, 96, 98, 100, 102 immersed in the effluent seawater 75 in intermittently spaced positions along the length LB of the basin 43. Each water quality sensor may comprise one or more detecting elements, similar to that described hereinbefore with reference to Fig. 2. Each water quality sensor 92, 94, 96, 98, 100, 102 is positioned to detect one or more parameters of the effluent seawater 75, and to send or transmit a signal to a control unit 110. The control unit 110 analyses the signals received from each of the water quality sensors 92, 94, 96, 98, 100, 102, and accordingly automatically controls the output from each of the blowers 276, 278, 280, 282, 284, 286, such that a suitable flow of oxygen containing gas is supplied to the effluent seawater 75 via each of the air distribution pipes 264, 266, 268, 270, 272, 274.

The chemical reactions occurring in wet scrubber tower 16 and in oxidation basin system 42 will now be described in more detail. The absorption of sulfur dioxide in interior 22 of wet scrubber tower 16, illustrated in Fig. 1, is assumed to occur according to the following reaction:

SO₂ (g) + H₂O = > HSO3⁻ (aq) + H⁺ (aq) [eq. 1.1a]

The bisulfite ions, HSO₃⁻, may, depending on the pH value of the effluent seawater, dissociate further to form sulfite ions, SO₃²⁻, in accordance with the following equilibrium reaction:

HSO₃⁻ (aq) <=> SO₃²⁻ (aq) + H⁺ (aq) [eq. 1.1b]

Hence, as an effect of the absorption of sulfur dioxide, the effluent seawater will have a lower pH value as an effect of the hydrogen ions, H⁺, generated in the absorption reaction, than that of the fresh seawater from the ocean 36, and will contain bisulfite and/or sulfite ions, HSO₃⁻ and SO₃²⁻ , respectively. Bisulfite and/or sulfite ions are oxygen demanding substances, and the release thereof to the ocean 36 is restricted.

In the oxidation basin system 42, oxygen gas, O₂(g), contained in the oxygen containing gas supplied via the oxygen supply system 47 is dissolved in the effluent seawater contained in the oxidation basin 43:

O₂ (g) < = > O₂ (aq) [eq. 1.2a]

The bisulfite and/or sulfite ions, HSO₃⁻ and/or SO₃²⁻ , are oxidized, at least partly, by reaction with the dissolved oxygen, in accordance with the following reactions:

HSO₃⁻ + H⁺ + ½ O₂ (aq) = > SO₄²⁻ + 2H⁺ [eq. 1.2b]

SO₃²⁻ + 2H⁺ + ½ O₂ (aq) = > SO₄²⁻ + 2H⁺ [eq. 1.2c]

Hence, as an effect of absorption of sulfur dioxide, and oxidation of the sulfite, hydrogen ions, H⁺, are generated in the effluent seawater. The seawater comprises calcium carbonate, CaCO₃, which functions as an alkali to react with and neutralize the hydrogen ions, H⁺. The neutralization could occur according to the following chemical reaction scheme. In a first step of the neutralization reaction, the carbonate ion, CO₃²⁻, reacts with one hydrogen ion, and forms a bicarbonate ion, HCO₃⁻:

CO₃²⁻ + H⁺ < = > HCO₃⁻ [eq. 2.1]

The formed bicarbonate ion, HCO₃⁻, may then react with a further hydrogen ion, H⁺, to form carbon dioxide, CO₂, in a dissolved state:

HCO₃⁻ + H⁺ < = > CO₂(aq) + H₂O [eq. 2.2]

Finally, the dissolved carbon dioxide, CO₂ (aq), is released to the atmosphere in gas form:

CO₂(aq) < = > CO₂(g) [eq. 2.3]

All of the neutralization reactions, [eq. 2.1 to 2.3], are equilibrium reactions. That means that the complete route, from carbonate, CO₃²⁻, to carbon dioxide, CO₂, in gas form will be rate limited by the slowest step. Of the neutralization reactions above, eq. 2.1 is the fastest, and eq. 2.2 is the slowest. Hence, eq. 2.2 will normally determine the rate at which hydrogen ions may be neutralized in the oxidation basin system 42.

The regulatory requirements regarding effluent water that can be returned to the ocean 36 often include the following parameters;
i) A sufficiently low amount of oxygen consuming substances (which is often referred to as COD, Chemical Oxygen Demand),
ii) A sufficiently high amount of oxygen, and
iii) A suitable pH

In a seawater scrubber 14, of the type disclosed in Fig. 1, the concentration of oxygen consuming substances, COD, normally correlates very well to the concentration of sulfite in the effluent seawater. Using water quality sensors 92, 94, 96, 98, 100, 102, each with a sulfite detecting element 104, an oxygen detecting element 106, and a pH detecting element 108, described hereinbefore with reference to Fig. 2, variations in the sulfite concentration, the oxygen concentration, and the pH, along the length LB of the oxidation basin 43 can be monitored and controlled.

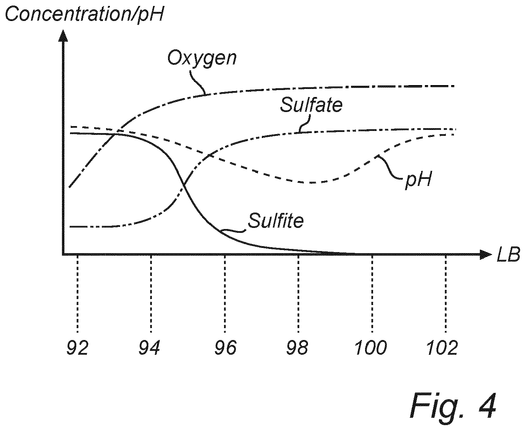

Fig. 4 illustrates an example of oxygen, sulfite and pH profiles from measurements taken along the length LB of the oxidation basin 43. While Fig. 4 and other figures refer to "Sulfite", it will be appreciated that "Sulfite" may include sulfite and/or bisulfite ions. Additionally, the concentration of sulfate, SO₄²⁻, is indicated. Sulfate is not measured as such, but is the end result of the oxidation of sulfite; hence the concentration of sulfate may be calculated from the measured concentration of sulfite. For reference, each of the water quality sensors 92, 94, 96, 98, 100, 102 positioned along the length LB of the oxidation basin 43 are labeled on the x-axis of Fig. 4.

As illustrated in Fig. 4, the concentration of oxygen, O₂(aq), dissolved in the effluent seawater increases rather quickly from water quality sensor 92 to water quality sensor 94, and by water quality sensor 96 a suitable concentration has been reached. As the concentration of dissolved oxygen, O₂(aq), in the effluent seawater 75 increases, the rate of oxidation of sulfite and/or bisulfite according to equations 1.2b and 1.2c increases. Hence, the concentration of sulfite rapidly decreases, between water quality sensor 94 and water quality sensor 98. As an effect of the oxidation of sulfite, the concentration of sulfate, SO₄²⁻, in the effluent water 75 increases. The oxidation of sulfite and/or bisulfite between water quality sensor 94 and water quality sensor 98 causes a formation of hydrogen ions, H⁺, according to eq. 1.2b. Such causes a reduction in the pH of the effluent water 75, between the second water quality sensor 94 and fourth water quality sensor 98. The neutralization reactions, eq. 2.1 to 2.3, continuously cause a neutralization of the formed hydrogen ions. Generally, there is a limited formation of hydrogen ions, H⁺, downstream of the fourth water quality sensor 98. However, the neutralization reactions, eq. 2.1 to 2.3, and in particular eq. 2.2, are not very fast, which means that some time is required before the pH reaches its desired level. Hence, the pH slowly increases, between the fourth water quality sensor 98 and the sixth water quality sensor 102.

The dissolution of oxygen in the effluent seawater, the oxidation of sulfite, and the neutralization of formed hydrogen ions to restore pH of the effluent seawater, are each governed by interactions between the chemical reactions. The control unit 110, depicted in Fig. 2, receives signals from each of the water quality sensors 92, 94, 96, 98, 100, 102 and controls each of the air distribution pipes 64, 66, 68, 70, 72, 74 to supply, in the appropriate position along the length LB of the oxidation basin 43, a suitable amount of oxygen containing gas for the effluent water leaving the basin 43 via the overflow pipe 48 to meet the regulatory requirements for oxygen content, COD and pH.

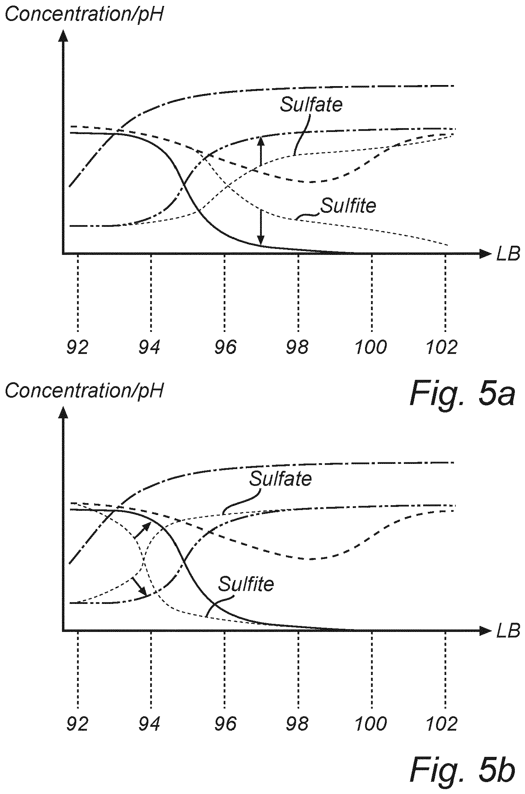

Fig. 5a illustrates an example in which sulfite detecting elements 104 in water quality sensors 92, 94, 96, 98, 100 register sulfite concentrations too high, as indicated by the broken lines in Fig. 5a. While the sulfite concentration measured by the sixth water quality sensor 102 may very well be within regulatory limits, there is a distinct risk that there may not be sufficient time to neutralize all hydrogen ions, H⁺, formed according to eq. 2.1 to 2.3, since hydrogen ion formation extends along almost the entire length LB of the oxidation basin 43. When the control unit 110 receives such information from water quality sensors 92, 94, 96, 98, 100, it may control the control valves 78, 80, 82, 84 of the second, third, fourth and fifth air distribution pipes 66, 68, 70, 72, respectively, to open to allow more oxygen to be supplied to the effluent water through the air distribution pipes. Optionally, the output of blower 44 may be increased. As an effect of an increased supply of oxygen, the sulfite concentration and the sulfate concentration are restored to their normal or desired values, as indicated in the illustration by means of arrows.

Fig. 5b illustrates an example in which sulfite detecting elements 104 of water quality sensors 92, 94, 96, 98, 100, 102 measure a sulfite concentration relatively low already at the third water quality sensor 96, as indicated by the broken lines in Fig. 5b. While the sulfite concentration, the oxygen concentration and the pH as measured by the sixth water quality sensor 102 are likely to be within the regulatory limits, there is a distinct risk that too much oxygen containing gas is being supplied to the effluent seawater, causing an increased amount of energy to be consumed by blower 44. When the control unit 110 receives such information from water quality sensors 92, 94, 96, 98, 100, 102, it may control the control valves 76, 78, 80 of the first, second, and third air distribution pipes 64, 66, 68, respectively, to close at least partly so less oxygen is supplied to the effluent water through the air distribution pipes. Optionally, also the output of the blower 44 is or may be reduced. As an effect of such a reduced supply of oxygen, the sulfite concentration and the sulfate concentration are restored to their normal or desired values, as indicated in the illustration by means of arrows.

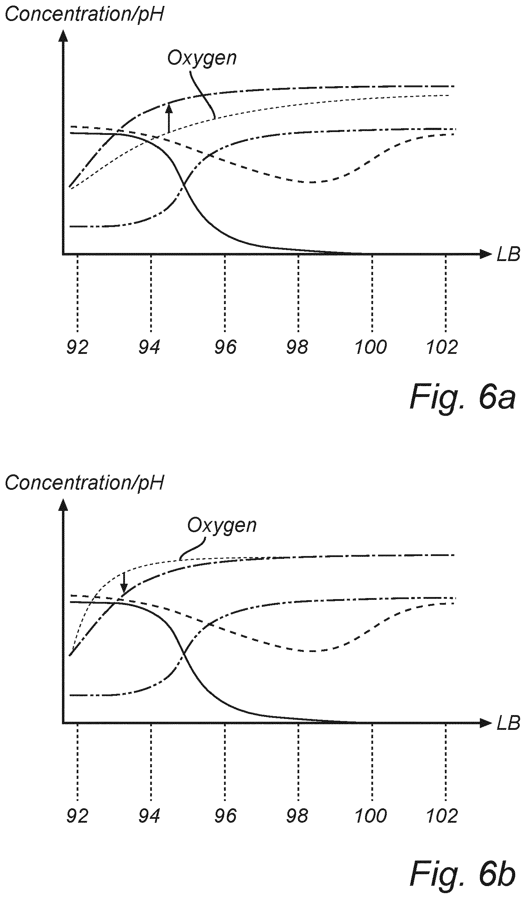

Fig. 6a illustrates an example where each oxygen detecting element 106 of water quality sensors 94, 96 registers an oxygen concentration that is too low, as indicated by the broken line in Fig. 6a. Such a low concentration of oxygen is likely to reduce the rate of sulfite oxidation, potentially causing a risk that the concentration of sulfite in the effluent seawater may exceed regulatory limits, and/or that the pH in the effluent seawater may get too low. When the control unit 110 receives such information from the water quality sensors 94, 96, it may control the control valves 76, 78 of first and second air distribution pipes 64, 66 to open to allow more oxygen to be supplied to the effluent water through those air distribution pipes. As an effect of such increased supply of oxygen, the oxygen concentration is restored to its normal value as indicated in the illustration of Fig. 6a by means of an arrow.

Fig. 6b illustrates an example where oxygen detecting elements 106 of water quality sensors 92, 94 register an oxygen concentration that is too high, as indicated by the broken line in Fig. 6b. Such a high concentration of oxygen indicates that too much of the oxygen containing gas is supplied to the effluent seawater, thus causing an increased amount of energy consumption by blower 44. When the control unit 110 receives such information from water quality sensors 92, 94, it may control the control valves 76, 78 of the first and second air distribution pipes 64, 66 to close, at least partly, such that less oxygen is supplied to the effluent water through those air distribution pipes. As an effect of such decreased supply of oxygen, the oxygen concentration is restored to its normal value, as indicated in the illustration of Fig. 6b by means of an arrow.

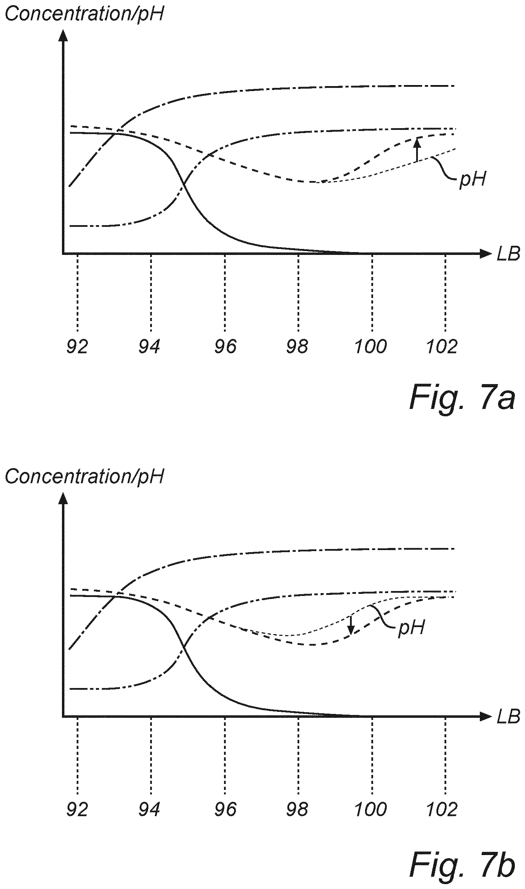

Fig. 7a illustrates an example in which the pH detecting elements 108 of water quality sensors 100, 102 register a pH value that is too low, as indicated by the broken lines in Fig. 7a. Such a low pH for the effluent seawater may not be acceptable for release to the ocean 36. When the control unit 110 receives such information from the water quality sensors 100, 102, it may control the control valves 84, 86 of fifth and sixth air distribution pipes 72, 74 to open, such that more air is supplied to the effluent water through those air distribution pipes. The supplied air has the effect of improving the gasification and subsequent removal of carbon dioxide, CO₂, from the effluent seawater according to eq. 2.3 set forth above. Such removal of gaseous CO₂ improves the speed of neutralization of hydrogen ions according to eq. 2.1 and 2.2 set forth above. As an effect of such increased supply of air, the pH value is restored to its normal value, as indicated in the illustration of Fig. 7a by means of an arrow.

Fig. 7b illustrates an example in which the pH detecting elements 108 of water quality sensors 98, 100 register a pH value that is at a suitable level for effluent water release to the ocean already at the fifth water quality sensor 100, as indicated by the broken lines in Fig. 7b. While the pH value is within the regulatory limits, there is a distinct risk that too much air is being supplied to the effluent seawater, causing an increased amount of energy to be consumed by blower 44. When the control unit 110 receives such information from water quality sensors 98, 100, it may control the control valves 82, 84 of fourth and fifth air distribution pipes 70, 72 to close, at least partly, such that less air is supplied to the effluent water through those air distribution pipes. As an effect of such reduced supply of oxygen, the pH value is restored to a more desirable normal value, as indicated in the illustration of Fig. 7b by means of an arrow.

Hence, as exemplified with reference to Figs. 5a, 5b, 6a, 6b, 7a, and 7b, control unit 110 controls, based on information/signals from the water quality sensors 92, 94, 96, 98, 100, 102, the amount of oxygen containing gas supplied through the individual air distribution pipes 64, 66, 68, 70, 72, 74, such that an optimum amount of oxygen is supplied in each respective location. Hence, a safe operation and a low energy cost oxidation basin system 42 may be achieved.

The control unit 110 may also be used for continuously supervising the sulfite concentration, and/or the oxygen concentration, and/or the pH value along the oxidation basin 43, and for adjusting the supply of oxygen containing gas supplied via the individual air distribution pipes 64, 66, 68, 70, 72, 74, accordingly. In this manner, process variations can be accounted for by adjusting the amount of air supplied via the various air distribution pipes. Such process variations include, for example, varying concentrations of sulfur dioxide in the flue gas generated by boiler 2, varying boiler loads, varying oxidation conditions due to, for example, varying temperatures, varying concentrations of oxidation catalyzing dust particles in the flue gas, and the like. It is also possible, as an alternative, to utilize control unit 110 only during start-up of the oxidation basin system 42, to tune a level of flow through the various distribution pipes 64, 66, 68, 70, 72, and 74. Furthermore, the setting of valves 76, 78, 80, 82, 84, 86, and/or the setting of the output of blowers 276, 278, 280, 282, 284, 286 could be made manually, as alternative to automatic control by control unit 110.

In the oxidation basin systems 42, 142 illustrated in Figs. 2 and 3 there are six consecutive air distribution pipes 64, 66, 68, 70, 72, 74 and six consecutive water quality sensors 92, 94, 96, 98, 100, 102. It will be appreciated that other numbers of air distribution pipes and water quality sensors may be used, depending on the length of the oxidation basin, and on the desired level or necessary accuracy of control required. Preferably, the oxidation basin system is provided with 2-20, more preferably 3-10, consecutive air distribution pipes, and 2-20, more preferably 3-10 consecutive water quality sensors. The number of consecutive air distribution pipes need not correspond to the number of water quality sensors. Hence, for example, an oxidation basin could be provided with six consecutive air distribution pipes, and four consecutive water quality sensors.

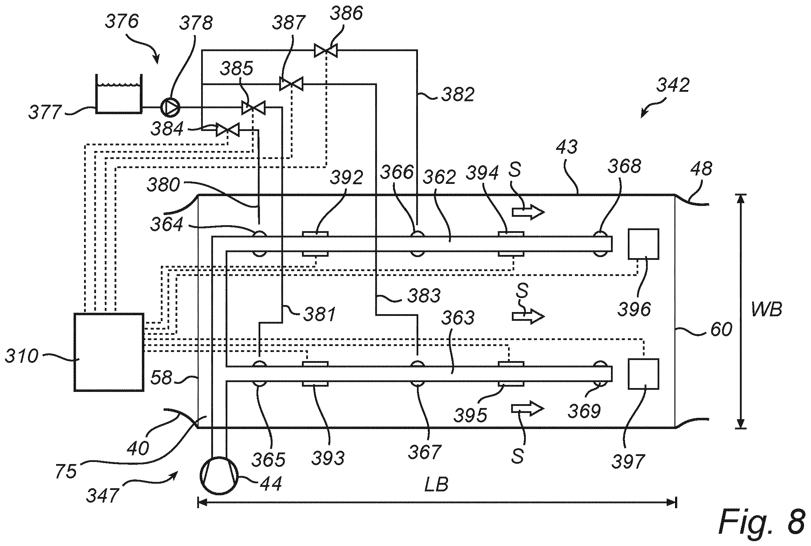

Fig. 8 is a schematic representation of an alternative oxidation basin system 342, from a top view. Features of the oxidation basin system 342 similar to those of oxidation basin system 42 have been given the same reference numerals. Effluent seawater is supplied to oxidation basin 43 of the oxidation basin system 342 via fluidly connected pipe 40 at first end 58, being an inlet end, of oxidation basin 43. The effluent seawater flows, generally horizontally as indicated by arrows S, along the length LB of the oxidation basin 43, from the first end 58 to a second end 60, and leaves basin 43 via fluidly connected overflow pipe 48.

The oxidation basin 43 has a considerable width WB. For this reason, the oxidation basin system 342 includes an oxygen supply system 347 comprising a first distribution duct 362 and a second distribution duct 363, each positioned in parallel with respect to the other and to extend along the length LB of the basin 43. The first distribution duct 362 comprises three air distribution pipes 364, 366, 368 arranged consecutively along the length LB of the basin 43, and the second distribution duct 363 comprises three air distribution pipes 365, 367, 369 arranged consecutively along the length LB of the basin 43. The air distribution pipes 364, 366, 368, 365, 367, 369 are of a similar design as the air distribution pipes 264, 266, 268 described hereinbefore with reference to Fig. 3. Air blown by blower 44 flows through distribution ducts 362, 363 to air distribution pipes 364, 366, 368, 365, 367, 369 and is mixed with effluent seawater.

Oxidation basin system 342 further comprises first, second, and third water quality sensors 392, 394, 396 immersed in the effluent seawater flowing through the basin 43. Water quality sensors 392, 394, 396 are arranged consecutively along first distribution duct 362, with the first water quality sensor 392 located in closest proximity to first end 58, the second water quality sensor 394 located downstream from first sensor 392, etc. Oxidation basin system 342 also comprises fourth, fifth, and sixth water quality sensors 393, 395, 397 immersed in the effluent seawater flowing through the basin 43. Water quality sensors 393, 395, 397 are arranged consecutively along second distribution duct 363, with the fourth water quality sensor 393 located in closest proximity to first end 58, the fifth water quality sensor 395 located downstream from fourth sensor 393, etc. Each water quality sensor may comprise one or more detecting elements, similar to those described hereinbefore with reference to Fig. 2. Each water quality sensor 392, 394, 396, 393, 395, 397 is positioned to detect one or more parameters of the effluent seawater, and for sending a signal/information to a control unit 310. Control unit 310 analyses the signals received from the respective water quality sensors 392, 394, 396, 393, 395, 397, and automatically controls a supply system 376 for supplying an oxidation enhancing substance to basin 43. Hence, in this embodiment a first oxidation enhancing substance in the form of an oxygen containing gas, supplied by oxygen supply system 347, is supplied along with a second oxidation enhancing substance, supplied via supply system 376.

The second oxidation enhancing substance could be an oxidation enhancing catalyst, such as iron, Fe, manganese, Mn, cobalt, Co, or copper, Cu. Furthermore, the oxidation enhancing substance may also be an oxidizing enzyme. An example of the latter is a sulfite oxidase type of enzyme. A sulfite oxidase may be prepared in accordance with the teachings of the article *"*Optimization of expression of human sulfite oxidase and its molybdenum domain" by CA Temple, TN Graf, and KV Rajagopalan, published in Arch. Biochem. Biophys. 2000 Nov 15;383(2):281-7.

Supply system 376 comprises a tank 377, in which an aqueous solution of an oxidation enhancing substance, such as iron or an oxidizing enzyme, is stored, and a pump 378 for transporting the solution to the basin 43. The pump 378 supplies solution to a first supply pipe 380, discharging enzyme adjacent to first distribution pipe 364, a second supply pipe 382, discharging enzyme adjacent to second distribution pipe 366, a third supply pipe 381, discharging enzyme adjacent to fourth distribution pipe 365, and a fourth supply pipe 383, discharging enzyme adjacent to fifth distribution pipe 367. Each supply pipe 380, 382, 381, 383 is provided with a respective control valve 384, 386, 385, 387 controlled by control unit 310.

The control unit 310 may utilize data from, for example, water quality sensors 392, 394, 396 to control the oxidation process occurring along the length LB of basin 43 in accordance with similar principles as those described hereinbefore with reference to Figs. 2-7b. A difference is that in oxidation basin system 342, the amount of air supplied to the oxidation basin system 342 need not be controlled to obtain the desired oxidation rate along the basin. Rather, the amount or use of oxidation catalyst, and/or the amount or use of oxidation enzyme, as the case may be, is controlled to obtain the desired oxidation rate. If, for example, a situation similar to that of Fig. 5a were to occur, control unit 310 may then control valve 384 to open to allow more of the catalyst, or enzyme, to be supplied via supply pipe 380 to a location in the proximity of first air distribution pipe 364.

According to a further alternative embodiment, the supply of oxygen containing gas from blower 44 may be controlled in accordance with principles described hereinbefore with reference to Figs. 2 and 3, and additionally the supply of catalyst and/or enzyme from tank 377 may be similarly controlled. Hence, the oxidation process occurring along the basin 43 could be controlled by controlling the supply of one or more oxidation enhancing substances. Examples of such oxidation enhancing substances include: an oxygen containing gas, an oxidation enhancing catalyst, and an oxidation enhancing enzyme.

Furthermore, control unit 310 could also compare data from parallel water quality sensors to monitor the system, control variations, and/or detect any malfunctions. Control unit 310 could, for example, compare a sulfite concentration measured by sensor 392 to a sulfite concentration measured by sensor 393. If, for example, a sulfite concentration measured by sensor 393 were higher than a sulfite concentration measured by sensor 392, then control unit 310 may cause valve 385 to open, such that more catalyst and/or enzyme is supplied via pipe 381 to a location in proximity to air distribution pipe 365, thereby increasing oxidation efficiency.

According to a still further embodiment, control unit 310 could compare data from parallel water quality sensors to monitor the system, control variations, and/or detect any malfunctions, by individually controlling the supply of air to first distribution duct 362 and second distribution duct 363. If, for example, a sulfite concentration measured by sensor 393 were higher than a sulfite concentration measured by sensor 392, then control unit 310 may cause an increased flow of air to be blown to second distribution duct 363 than that blown to first distribution duct 362. Such control could, for example, be carried out by arranging separate blowers for each of the distribution ducts, 362, 363, or by arranging valves on the distribution ducts 362, 363. Furthermore, control unit 310 could compare data from parallel water quality sensors to monitor the system, control variations, and/or detect any malfunctions, by individually controlling the supply of air to air distribution pipes 364, 366, 368, 365, 367, 369. If, for example, a sulfite concentration measured by sensor 395 were higher than a sulfite concentration measured by sensor 394, then control unit 310 may cause an increased flow of air to be blown to air distribution pipe 367 than that blown to air distribution pipe 366. Such control could, for example, be carried out by arranging valves on the air distribution pipes 364, 366, 368, 365, 367, 369 in a manner similar to that illustrated in Fig. 2, or by arranging separate blowers for each of the air distribution pipes 364, 366, 368, 365, 367, 369 in a manner similar to that illustrated in Fig. 3. Still further, controlling the supply of a first oxidation enhancing substance in the form of air at a particular location in the basin could be combined with controlling the supply of a second oxidation enhancing substance, e.g. an oxidation enhancing catalyst, to that same location in the basin. Such allows for more options to control the system, and allows for the possibility of very vigorous control by, for example, simultaneously increasing the flow of air and the supply of an oxidation enhancing catalyst to cause a very powerful increase in oxidation in a specific location.

A method of controlling the treatment of effluent seawater comprises:
- flowing effluent seawater along an oxidation basin 43,
- supplying an oxygen containing gas as an oxidation enhancing substance in a first supply location and in a second supply location to oxidize at least a portion of a content of sulfite and/or bisulfite of the effluent seawater,
- measuring at least one parameter relating to the oxidation of sulfite and/or bisulfite in a first measurement location downstream of the first supply location, and in a second measurement location downstream of the second supply location, and
- controlling, based on the parameters measured in the first and second measurement locations, a first amount of oxidation enhancing substance supplied in one of the first and second supply locations independently from a second amount of oxidation enhancing substance supplied in the other one of the first and second supply locations.

Hence, according to this method, it is possible to control only the amount of oxidation enhancing substance supplied in the first supply location, with the amount of oxidation enhancing substance supplied in the second supply location held constant, or to control only the amount of oxidation enhancing substance supplied in the second supply location, with the amount of oxidation enhancing substance supplied in the first supply location held constant, or to control both the amount of oxidation enhancing substance supplied in the first supply location and the amount of oxidation enhancing substance supplied in the second supply location.

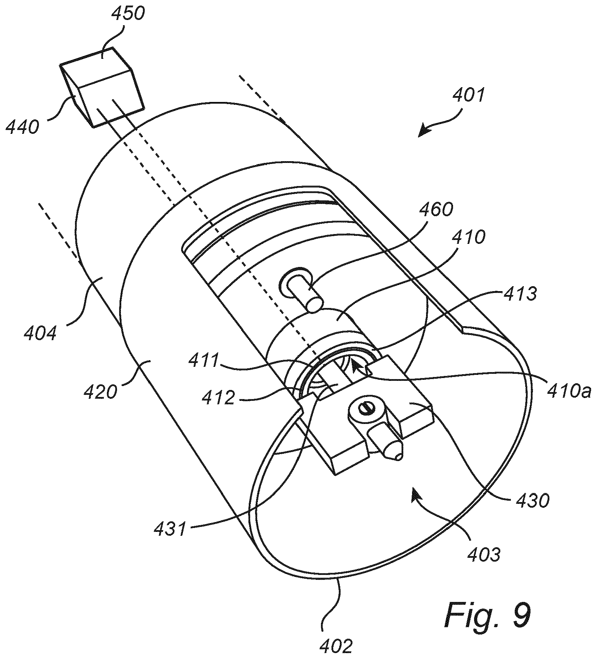

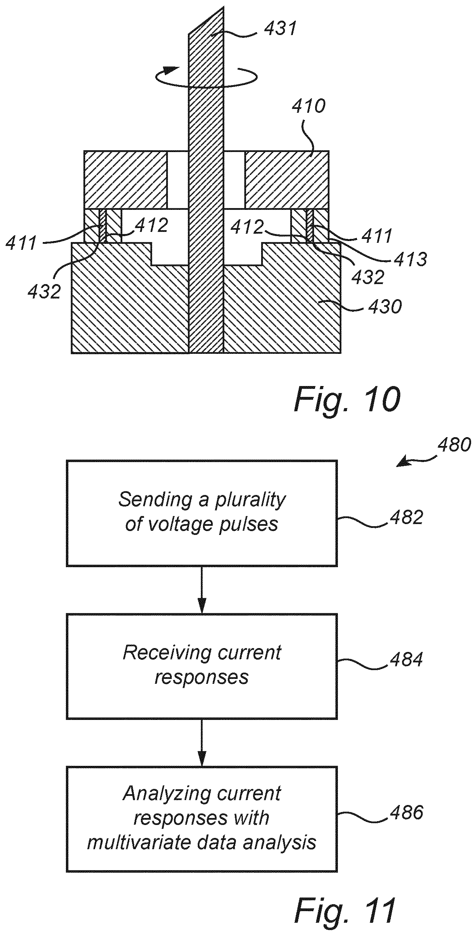

A sulphite sensor 401 which may be utilized as a sulfite detecting element 104 is illustrated in a perspective view in Fig. 9 and in a schematic cross-sectional side view in Fig. 10. The sulphite sensor 401 comprises a base section 404, and a cover 402 that forms sides of a space 403 for sulphite detection. A sensor head 410 is located in the space 403. The sensor head 410 is formed as a tube extending into the space 403. At an axial end portion 413 of the sensor head 410, a first electrode 411 having the form of a platinum ring is provided. A surface 412 of the first electrode 411 is level with the axial end portion 413 of the sensor head 410.

A shaft 431 extends through interior 410a of the tube shaped sensor head 410. The shaft 431 is rotated by an electric motor (not shown). The shaft 431 is coupled to a grinding unit 430. The grinding unit 430 has a surface 432, which is best shown in Fig. 10, adapted to abut the surface 412 of the first electrode 411. The shaft 431 rotates the grinding unit 430 such that the surface 432 of the grinding unit 430 grinds/cleans the surface 412 of the first electrode 411. The grinding unit 430 rotates in contact with the surface 412 of the first electrode 411 at a speed of 2-40 rpm, preferably at a speed of 15 rpm. The grinding unit 430 is preferably made of a ceramic material based on e.g. silicon carbide or silicon nitride.

The sulphite sensor 401 further comprises a second electrode 420. The second electrode 420 is preferably of a metal, such as steel or the like. The second electrode 420 is located at a distance from the first electrode 411. In the illustrated embodiment, the second electrode 420 is constituted by the metal cover 402.

A control unit 440 is arranged in the sulphite sensor 401 or connected to the sulphite sensor 401 and is adapted to send voltage pulses through the substance occupying the space between the first electrode 411 and the second electrode 420. When the sulphite sensor 401 is submerged into a substance, the voltage pulses enter the substance via the first electrode 411. The second electrode 420 is adapted to receive current responses generated by said voltage pulses and pass the current responses back to the control unit 440. The control unit 440 receives and analyzes using an analyzing unit 450 the current responses and calculates a concentration of sulphite in the substance using a multivariate data analysis. By using multivariate data analysis in the analyzing unit 450, mathematical models from samples with known sulphite concentrations is used for creating a prediction model that is used for determining the sulphite concentration in an unknown substance.

Data from voltammetric measurements are often difficult to interpret. Each measurement consists of a number of variables. Multivariate data analyzing methods, such as principal component analysis (PCA) and projection to latent structure (PLS), as is known from, for example,: Wold,S., Esbensen, K. and Geladi, P. "Principal component analysis: A tutorial." Chemometrics and Intelligent Laboratory Systems 2, 37-52, 1987.; and from: S. Wold, M. Sjöström and L. Eriksson "PLS-regression: a basic tool of chemometrics" Chemometrics and Intelligent Laboratory Systems, 58 (2001) 109-130, have shown to be useful. PCA is a mathematical tool, which describes the variance in experimental data. A vector is calculated which describes the direction of the largest variance in experimental data, that is the direction that describes the largest differences between observations. This vector is called the first principal component. The second principal component is orthogonal to and thus independent of the first principal component. Further principal components can be calculated in a similar way, until most of the observations are explained. A new matrix, as defined by the principal components is then formed, and the data set is considerably reduced, depending on the significance of the different principal components, but in many cases only to two dimensions. The loading vectors describe the direction of the principal components in relation to the original variables, and the score vectors, describe the direction of the principal components in relation to the observations. Thus, a score plot can be made, showing the relationships between the original samples and how much they influence the system. Thus, a score plot shows the relationships between the experiments, and groupings of them can be used for classification.

PLS is used to make models from calibration sets of data. It is a linear method, in which PCA is performed on both the X-data (the voltammogram) and the Y-data (the concentrations). Then a linear regression is performed on each PC between the datasets and the Y-data, giving a regression model. This model can be used to predict values from the voltammograms.

Further information regarding multivariate data analysis may be found in I.T. Jolliffe "Principle Component Analysis" Springer-Verlag, New York inc. (1986) ISBN 0-387-96269-7, or K.R. Beebe, R.J. Pell and M.B. Seasholtz "Chemometrics - A practical guide" John Wiley & Sons Inc. (1998) ISBN 0-471-12451-6.

In one embodiment, the sulphite sensor 401 further comprises a temperature sensor 460 for measuring the temperature of the substance.

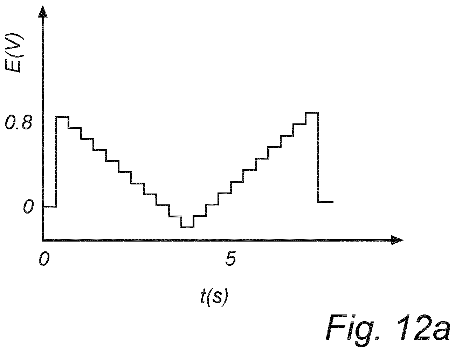

Fig. 11 is a flow chart of a method 480 for measuring a concentration of sulphite in a substance. The substance may be provided in a gas cleaning process. In a step 482, a plurality of voltage pulses is sent through the first electrode 411. The first electrode 411 is in contact with the substance. Voltage pulses are sent from the control unit 440 by the first electrode 411 and the second electrode 420 through the substance as a stepwise increasing or decreasing voltage level as shown in Fig. 12a. A staircase pattern of the voltage level sent through the first electrode 411 is formed. Each step involves increasing or decreasing the voltage level by, preferably, about 0.05 V. In an example of the method, the voltage level sent through the substance as voltage pulses is, in a stepwise manner, increased from a voltage level of -1.0 V to a voltage level of 1.0 V in steps of 0.05 V. In a further example, illustrated in fig. 12a, the voltage level is first decreased from 0.8 V and down to -0.1 V, in steps of 0.05 V, and is then increased from 0.1 V and up to 0.8 V, in steps of 0.05 V.

In a step 484, current responses are received, which current responses are generated by the voltage pulses sent by the first electrode 411 to the second electrode 420. The current responses are received by the second electrode 420. The second electrode 420 is also in contact with the substance. Each step of increasing or decreasing the voltage level generates a new current response in the second electrode 420.

In a final step 486, the current responses are analyzed using a multivariate data analysis. The concentration of sulphite in the substance may thereby be measured based on the current responses. According to one embodiment, all of the plurality of current responses is used for the measurement of sulphite concentration in the substance. In one embodiment, the current response is analyzed after each sent voltage pulse. Alternatively, a series of voltage pulses are sent, generating a series of current responses, before multivariate data analysis is performed on the series of current responses.

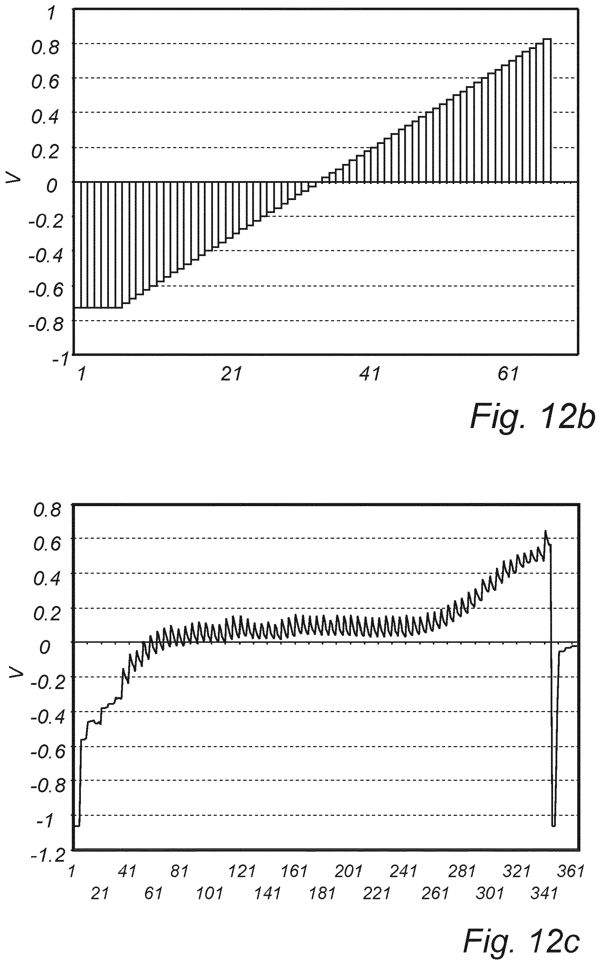

Fig. 12b further shows an example simulation of voltage pulses in a staircase pattern. The voltage level varies over time from approximately -0.75 V to approximately 0.8 V. The values on the x axis represent number of voltage pulses. Fig. 12c shows corresponding current responses as an outgoing voltage from an electronic circuit. Information from the current responses is used for estimating the sulphite level in the substance, using multivariate data analysis. Each voltage pulse as shown in Fig. 12b corresponds to five measured voltage values in Fig. 12c. Hence, in the example shown in Figs. 12b and 12c, the response of each voltage pulse is measured five times during each voltage pulse. The values on the x axis of Fig. 12c represent number of measurements. It will be appreciated that other types of sulphite sensors may also utilized as the sulfite detecting element 104.

It will be appreciated that numerous modifications of the embodiments described above are possible within the scope of the appended claims.

Hereinbefore, it has been described that each water quality sensor 92, 94, 96, 98, 100, 102 is provided with a sulfite detecting element 104, an oxygen detecting element 106, and a pH detecting element 108. It will be appreciated that other arrangements are also possible. In accordance with one embodiment, the water quality sensors could be provided with only a sulfite concentration detecting element 104, or only an oxygen concentration detecting element 106, or only a pH detecting element 108. According to a still further embodiment, the water quality sensors of an oxidation basin system could be provided with different numbers of detecting elements. Hence, for example, some of the water quality sensors could be provided with all three detecting elements 104, 106, 108, while some water quality sensors could be provided with only one or only two such detecting elements.

Hereinbefore it has been described that the supply of oxygen containing gas is controlled individually for each of the consecutive air distribution pipes 64, 66, 68, 70, 72, 74. It will be appreciated that it is also possible to individually control just one or some of the consecutive air distribution pipes 64, 66, 68, 70, 72, 74. For example, it would be possible to individually control only the second air distribution pipe 66, and to allow the rest of the air distribution pipes 64, 68, 70, 72, 74 to operate with a fixed flow.

Hereinbefore, it has been described that an oxidation enhancing substance in the form of a catalyst or an enzyme may be supplied in the form of an aqueous solution. It will be appreciated that such substances could be supplied in other forms as well. For example, the substance could be supplied in solid form. A further option is to cause release of, for example, iron, Fe, as an oxidation catalyst, from an iron plate electrode immersed in the oxidation basin. In such a case, the iron plates are connected to a source of power, and the voltage or current supplied between the iron plate electrode and a counter electrode is controlled continuously or periodically to control the release of iron to the effluent seawater.

Hereinbefore it has been described that the oxidation basin system may comprise, for example, 2-20 consecutive oxygen distributing pipes, and 2-20 consecutive water quality sensors. It will be appreciated that the oxidation basin system may comprise any number of parallel or nonparallel distribution ducts 362, 363, and parallel or nonparallel water quality sensors, to suit the width WB of the oxidation basin.

To summarize, a seawater oxidation basin system 42 for treating effluent seawater comprises, inter alia:
a first supply pipe 64 for distributing oxidation enhancing substance in the effluent seawater 75,
a second supply pipe 66 for distributing oxidation enhancing substance in the effluent seawater 75, and
a control device 76, 78 for controlling a first amount of an oxygen containing gas as an oxidation enhancing substance supplied by one of the first and second supply pipes 64, 66 independently from a second amount of an oxygen containing gas as an oxidation enhancing substance supplied by the other one of the first and second supply pipes 64, 66.

The use of the terms first, second, etc. do not denote any order or importance, but rather the terms first, second, etc. are used to distinguish one element from another.

## Claims

1. A method of controlling treatment of an effluent seawater generated in removing sulfur dioxide from a process gas by contacting the process gas containing sulfur dioxide with seawater comprises:
flowing effluent seawater along an oxidation basin (43),
supplying an oxygen containing gas as an oxidation enhancing substance in a first supply location and in a second supply location to oxidize at least a portion of a content of sulfite and/or bisulfite in the effluent seawater,
measuring at least one parameter relating to oxidation of sulfite and/or bisulfite in a first measurement location downstream of the first supply location, and in a second measurement location downstream of the second supply location, and
controlling, based on the parameter measured in the first and second measurement locations, a first amount of oxidation enhancing substance supplied in one of the first and second supply locations independently from a second amount of oxidation enhancing substance supplied in the other one of the first and second supply locations.

2. A method according to claim 1, wherein the second supply location is downstream, with regard to the effluent seawater flow along the oxidation basin (43), of the first supply location.

3. A method according to any one of the preceding claims, wherein the oxidation enhancing substance further comprises at least one of: an oxidation enhancing catalyst, and an oxidation enhancing enzyme.

4. A method according to any one of the preceding claims, further comprising measuring at least one parameter relating to oxidation of sulfite and/or bisulfite selected from the group of parameters consisting of: sulfite concentration, oxygen concentration, and pH.

5. A method according to any one of the preceding claims, further comprising independently controlling a first amount of oxidation enhancing substance supplied in the first supply location and independently controlling a second amount of oxidation enhancing substance supplied in the second supply location.

6. A method according to any one of the preceding claims, further comprising supplying oxidation enhancing substance in at least first, second, third, and fourth supply locations along the length of oxidation basin (43), and measuring at least one parameter in al least first, second, third and fourth measurement locations along the length of oxidation basin (43), each downstream of a supply location.

7. A method according to any one of the preceding claims, further comprising measuring a concentration of sulfite and/or bisulfile in the first measurement location, and controlling a first amount of oxidation enhancing substance supplied in the first supply location based on the measured concentration of sulfite and/or bisulfite.

8. A seawater oxidation basin system (42) for treating an effluent seawater generated in a wet scrubber (14) in which a process gas is brought into contact with seawater for removal of sulfur dioxide from said process gas, **characterised in** the oxidation basin system (42) comprising an oxidation basin (43) along which the effluent seawater flows,
a first supply pipe (64; 264; 380) positioned in the oxidation basin (43) for distributing oxidation enhancing substance in the effluent seawater (75),
a second supply pipe (66; 266; 381) positioned in the oxidation basin (43) for distributing oxidation enhancing substance in the effluent seawater (75), at least one control device (76, 78; 276; 278; 384, 385) for controlling a first amount of an oxygen containing gas as an oxidation enhancing substance supplied by one of the first and
second supply pipes (64, 66; 264, 266; 380, 381) independently from a second amount of an oxygen containing gas as an oxidation enhancing substance supplied by the other one of the first and second supply pipes (64, 66; 264, 266; 380, 381),
a first water quality sensor (92) positioned in the oxidation basin (43) downstream of the first supply pipe (64; 264; 380) for measuring at least one parameter relating to the oxidation of sulfite and/or bisulfite in the oxidation basin, and
a second water quality sensor (94) positioned in the oxidation basin (43) downstream of the second supply pipe (66; 266; 381) for measuring at least one parameter relating to the oxidation of sulfite and/or bisulfite in the oxidation basin (43),
further comprising a control unit (110, 310) for controlling, based on parameters measured by the first and second water quality sensors (92, 94), the first amount of oxidation enhancing substance supplied by one of the first and second supply pipes
(64, 66; 264, 266; 380; 381) independently from a second amount of oxidation enhancing substance supplied by the other one of the first and second supply pipes (64, 66; 264, 266; 380, 381).

9. An oxidation basin system according to claim 8, wherein
the second supply pipe (66; 266) is positioned in the oxidation basin (43) 20 downstream of the first supply pipe (64; 264).

10. An oxidation basin system according to any one of claims 8-9, wherein each of the first and second water quality sensors (92, 94) further comprises
at least one detecting element (104, 108) selected from the group
consisting of: sulfite detecting elements (104, 401), and pH detecting elements (108).

11. An oxidation basin system according to any one of claims 8-10,
wherein the first and second supply pipes (64, 66; 380, 382) are provided with individual control valves (76, 78; 384, 386) for controlling the amount of oxidation enhancing substance supplied by the respective supply pipe (64, 66; 380, 382).

12. An oxidation basin system according to any one of claims 8-11,
wherein the first and second supply pipes (264, 266) are connected to individual blowing devices (276, 278) for controlling an amount of oxygen containing gas supplied t11 rough the respective supply pipe (264, 266).

13. An oxidation basin system according to any one of claims 8-12,
wherein the oxidation basin system (42; 142) comprises 3-10 consecutive supply pipes (64, 66, 68, 70, 72, 74; 264, 266, 268, 270, 272, 274), and 3-10 consecutive water quality sensors (92, 94, 96, 98, 100, 102).

14. A seawater based process gas cleaning system comprising a wet scrubber (14) in which a process gas is brought into contact with seawater for removal of sulfur dioxide from said process gas, and an oxidation basin system (42) according to any one of claims 8 to13 for treating effluent seawater (75) generated in the wet scrubber (14) in conjunction with the removal of sulfur dioxide from the process gas.

## Patentansprüche

1. Verfahren zum Regeln einer Behandlung eines Abwassers aus Meerwasser, das durch Entfernen von Schwefeldioxid aus einem Prozessgas generiert wird, indem das Prozessgas, das Schwefeldioxid enthält, mit Meerwasser in Kontakt gebracht wird, umfassend:
Strömen von Abwasser aus Meerwasser entlang eines Oxidationsbeckens (43),
Zuführen eines Sauerstoffs, der Gas enthält, als eine oxidationsfördernde Substanz, an einer ersten Zuführstelle und an einer zweiten Zuführstelle, um mindestens einen Teil eines Gehalts von Sulfit und/oder Bisulfit in dem Abwasser aus Meerwasser zu oxidieren,
Messen mindestens eines Parameters, der sich auf eine Oxidation von Sulfit und/oder Bisulfit bezieht, an einer ersten Messstelle stromabwärts der ersten Zuführstelle, und an einer zweiten Messstelle stromabwärts der zweiten Zuführstelle, und
Regeln, auf Grundlage des an den ersten und zweiten Messstellen gemessenen Parameters, einer ersten Menge von oxidationsfördernder Substanz, die an einer von den ersten und zweiten Zuführstellen zugeführt wird, unabhängig von einer zweiten Menge von oxidationsfördernder Substanz, die an der anderen von den ersten und zweiten Zuführstellen zugeführt wird.

2. Verfahren nach Anspruch 1, wobei die zweite Zuführstelle sich bezüglich der Strömung des Abwassers aus Meerwasser entlang des Oxidationsbeckens (43) stromabwärts der ersten Zuführstelle befindet.

3. Verfahren nach einem der vorstehenden Ansprüche, wobei die oxidationsfördernde Substanz weiter mindestens eines der Folgenden umfasst: einen oxidationsfördernden Katalysator und ein oxidationsförderndes Enzym.

4. Verfahren nach einem der vorstehenden Ansprüche, weiter umfassend ein Messen mindestens eines Parameters, der sich auf eine Oxidation von Sulfit und/oder Bisulfit bezieht, ausgewählt aus der Gruppe von Parametern bestehend aus: Sulfitkonzentration, Sauerstoffkonzentration und pH-Wert.

5. Verfahren nach einem der vorstehenden Ansprüche, weiter umfassend ein unabhängiges Regeln einer ersten Menge von oxidationsfördernder Substanz, die an der ersten Zuführstelle zugeführt wird, und unabhängiges Regeln einer zweiten Menge von oxidationsfördernder Substanz, die an der zweiten Zuführstelle zugeführt wird.

6. Verfahren nach einem der vorstehenden Ansprüche, weiter umfassend ein Zuführen von oxidationsfördernder Substanz an mindestens ersten, zweiten, dritten und vierten Zuführstellen entlang der Länge des Oxidationsbeckens (43), und Messen mindestens eines Parameters an mindestens ersten, zweiten, dritten und vierten Messstellen entlang der Länge des Oxidationsbeckens (43), die jeweils stromabwärts einer Zuführstelle liegen.

7. Verfahren nach einem der vorstehenden Ansprüche, weiter umfassend ein Messen einer Konzentration von Sulfit und/oder Bisulfit an der ersten Messstelle, und Regeln einer ersten Menge von oxidationsfördernder Substanz, die an der ersten Zuführstelle zugeführt wird, auf Grundlage der gemessenen Konzentration von Sulfit und/oder Bisulfit.

8. Meerwasseroxidationsbeckensystem (42), zur Behandlung eines Abwassers aus Meerwasser, das in einem Nasswäscher (14) generiert wird, in welchem ein Prozessgas zur Entfernung von Schwefeldioxid aus dem Prozessgas in Kontakt mit Meerwasser gebracht wird, **dadurch gekennzeichnet, dass** das Oxidationsbeckensystem (42) umfasst:
ein Oxidationsbecken (43), entlang dessen das Abwasser aus Meerwasser strömt,
eine erste Zuführleitung (64; 264; 380), die in dem Oxidationsbecken (43) positioniert ist, um oxidationsfördernde Substanz in dem Abwasser aus Meerwasser (75) zu verteilen,
eine zweite Zuführleitung (66; 266; 381), die in dem Oxidationsbecken (43) positioniert ist, um oxidationsfördernde Substanz in dem Abwasser aus Meerwasser (75) zu verteilen,
mindestens eine Regelungsvorrichtung (76, 78; 276; 278; 384, 385) zum Regeln einer ersten Menge von sauerstoffhaltigem Gas als eine oxidationsfördernde Substanz, die von einer der ersten und zweiten Zuführleitungen (64, 66; 264, 266; 380, 381) zugeführt wird, unabhängig von einer zweiten Menge von sauerstoffhaltigem Gas als eine oxidationsfördernde Substanz, die von der anderen der ersten und zweiten Zuführleitungen (64, 66; 264, 266; 380, 381) zugeführt wird,
einen ersten Wasserqualitätssensor (92), der in dem Oxidationsbecken (43) stromabwärts der ersten Zuführleitung (64; 264; 380) positioniert ist, zum Messen mindestens eines Parameters, der sich auf die Oxidation von Sulfit und/oder Bisulfit in dem Oxidationsbecken bezieht, und
einen zweiten Wasserqualitätssensor (94), der in dem Oxidationsbecken (43) stromabwärts der zweiten Zuführleitung (66; 366; 381) positioniert ist, zum Messen mindestens eines Parameters, der sich auf die Oxidation von Sulfit und/oder Bisulfit in dem Oxidationsbecken (43) bezieht,
weiter umfassend eine Regelungseinheit (110, 310) zum Regeln, auf Grundlage der von den ersten und zweiten Wasserqualitätssensoren (92, 94) gemessenen Parameter, der ersten Menge von oxidationsfördernder Substanz, die von einer der ersten und zweiten Zuführleitungen (64, 66; 264, 266; 380; 381) zugeführt wird, unabhängig von einer zweiten Menge von oxidationsfördernder Substanz, die von der anderen der ersten und zweiten Zuführleitungen (64, 66; 264, 266; 380; 381) zugeführt wird.

9. Oxidationsbeckensystem nach Anspruch 8, wobei die zweite Zuführleitung (66, 266) in dem Oxidationsbeckens (43) stromabwärts der ersten Zuführleitung (64, 264) positioniert ist.

10. Oxidationsbeckensystem nach einem der Ansprüche 8-9, wobei jeder der ersten und zweiten Wasserqualitätssensoren (92, 94) weiter mindestens ein detektierendes Element (104, 108) umfasst, das ausgewählt ist aus der Gruppe bestehend aus: sulfitdetektierenden Elementen (104, 401) und pH-Wertdetektierenden Elementen (108).

11. Oxidationsbeckensystem nach einem der Ansprüche 8-10, wobei die ersten und zweiten Zuführleitungen (64, 66; 380, 382) mit einzelnen Regelungsventilen (76, 78; 384, 386) zum Regeln der Menge von oxidationsfördernder Substanz, die von der entsprechenden Zuführleitung (64, 66; 380, 382) zugeführt wird, versehen sind.

12. Oxidationsbeckensystem nach einem der Ansprüche 8-11, wobei die ersten und zweiten Zuführleitungen (264, 266) mit einzelnen Gebläsevorrichtungen (276, 278) zum Regeln einer Menge von sauerstoffhaltigem Gas, die durch die entsprechende Zuführleitung (264, 266) zugeführt wird, verbunden sind.

13. Oxidationsbeckensystem nach einem der Ansprüche 8-12, wobei das Oxidationsbeckensystem (42; 142) 3-10 auf einander folgende Zuführleitungen (64, 66, 68, 70, 72, 74; 264, 266, 268, 270, 272, 274) und 3-10 aufeinander folgende Wasserqualitätssensoren (92, 94, 96, 98, 100, 102) umfasst.

14. Meerwasserbasiertes Prozessgasreinigungssystem, umfassend einen Nasswäscher (14), in welchem ein Prozessgas zur Entfernung von Schwefeldioxid aus dem Prozessgas in Kontakt mit Meerwasser gebracht wird, und ein Oxidationsbeckensystem (42) nach einem der Ansprüche 8 bis 13 zur Behandlung von Abwasser aus Meerwasser (75), das in dem Nasswäscher (14) generiert wird, in Zusammenhang mit der Entfernung von Schwefeldioxid aus dem Prozessgas.

## Revendications

1. Procédé de commande du traitement d'un effluent d'eau de mer généré lors de l'élimination du dioxyde de soufre à partir d'un gaz de procédé par la mise en contact du gaz de procédé contenant du dioxyde de soufre avec de l'eau de mer comprenant :
l'écoulement d'un effluent d'eau de mer le long d'un bassin d'oxydation (43),
l'introduction d'un gaz contenant de l'oxygène en tant que substance de renforcement d'oxydation dans un premier emplacement d'alimentation et dans un deuxième emplacement d'alimentation pour oxyder au moins une partie d'une teneur en sulfite et/ou en bisulfite dans l'effluent d'eau de mer,
la mesure d'au moins un paramètre relatif à l'oxydation du sulfite et/ou du bisulfite dans un premier emplacement de mesure en aval du premier emplacement d'alimentation, et dans un deuxième emplacement de mesure en aval du deuxième emplacement d'alimentation, et
la commande, sur la base du paramètre mesuré dans les premier et deuxième emplacements de mesure, d'une première quantité de substance de renforcement d'oxydation introduite dans l'un parmi les premier et deuxième emplacements d'alimentation indépendamment d'une deuxième quantité de substance de renforcement d'oxydation introduite dans l'autre parmi les premier et deuxième emplacements d'alimentation.

2. Procédé selon la revendication 1, dans lequel le deuxième emplacement d'alimentation est en aval, en ce qui concerne l'écoulement de l'effluent d'eau de mer le long du bassin d'oxydation (43), du premier emplacement d'alimentation.

3. Procédé selon l'une quelconque des revendications précédentes, dans lequel la substance de renforcement d'oxydation comprend en outre au moins l'un parmi : un catalyseur de renforcement d'oxydation, et une enzyme de renforcement d'oxydation.

4. Procédé selon l'une quelconque des revendications précédentes, comprenant en outre la mesure d'au moins un paramètre relatif à l'oxydation du sulfite et/ou du bisulfite sélectionné dans le groupe de paramètres constitué : de la concentration en sulfite, de la concentration en oxygène, et du pH.

5. Procédé selon l'une quelconque des revendications précédentes, comprenant en outre la commande indépendante d'une première quantité de substance de renforcement d'oxydation introduite dans le premier emplacement d'alimentation et la commande indépendante d'une deuxième quantité de substance de renforcement d'oxydation introduite dans le deuxième emplacement d'alimentation.

6. Procédé selon l'une quelconque des revendications précédentes, comprenant en outre l'introduction d'une substance de renforcement d'oxydation dans au moins un premier, un deuxième, un troisième, et un quatrième emplacement d'alimentation le long de la longueur du bassin d'oxydation (43), et la mesure d'au moins un paramètre dans au moins un premier, un deuxième, un troisième et un quatrième emplacement de mesure le long de la longueur du bassin d'oxydation (43), chacun en aval d'un emplacement d'alimentation.

7. Procédé selon l'une quelconque des revendications précédentes, comprenant en outre la mesure d'une concentration en sulfite et/ou en bisulfite dans le premier emplacement de mesure, et la commande d'une première quantité de substance de renforcement d'oxydation introduite dans le premier emplacement d'alimentation sur la base de la concentration en sulfite et/ou en bisulfite mesurée.

8. Système de bassin d'oxydation d'eau de mer (42) pour traiter un effluent d'eau de mer généré dans un laveur par voie humide (14) dans lequel un gaz de procédé est mis en contact avec de l'eau de mer pour éliminer du dioxyde de soufre à partir dudit gaz de procédé, **caractérisé en ce que** le système de bassin d'oxydation (42) comprend
un bassin d'oxydation (43) le long duquel l'effluent d'eau de mer s'écoule,
un premier tuyau alimentation (64; 264; 380) positionné dans le bassin d'oxydation (43) pour distribuer une substance de renforcement d'oxydation dans l'effluent d'eau de mer (75),
un second tuyau d'alimentation (66; 266; 381) positionné dans le bassin d'oxydation (43) pour distribuer une substance de renforcement d'oxydation dans l'effluent d'eau de mer (75),
au moins un dispositif de commande (76, 78; 276; 278; 384, 385) pour commander une première quantité d'un gaz contenant de l'oxygène en tant que substance de renforcement d'oxydation introduite par l'un parmi les premier et second tuyaux d'alimentation (64, 66; 264, 266; 380, 381) indépendamment d'une seconde quantité d'un gaz contenant de l'oxygène en tant que substance de renforcement d'oxydation introduite par l'autre parmi les premier et second tuyaux d'alimentation (64, 66 ; 264, 266 ; 380, 381),
un premier capteur de qualité d'eau (92) positionné dans le bassin d'oxydation (43) en aval du premier tuyau d'alimentation (64; 264; 380) pour mesurer au moins un paramètre relatif à l'oxydation du sulfite et/ou du bisulfite dans le bassin d'oxydation, et
un second capteur de qualité d'eau (94) positionné dans le bassin d'oxydation (43) en aval du second tuyau d'alimentation (66; 266; 381) pour mesurer au moins un paramètre relatif à l'oxydation du sulfite et/ou du bisulfite dans le bassin d'oxydation (43),
comprenant en outre une unité de commande (110, 310) pour commander, sur la base des paramètres mesurés par les premier et second capteurs de qualité d'eau (92, 94), la première quantité de substance de renforcement d'oxydation introduite par l'un parmi les premier et second tuyaux d'alimentation (64, 66; 264, 266; 380, 381) indépendamment d'une seconde quantité de substance de renforcement d'oxydation introduite par l'autre parmi les premier et second tuyaux d'alimentation (64, 66; 264, 266; 380, 381).

9. Système de bassin d'oxydation selon la revendication 8, dans lequel
le second tuyau d'alimentation (66; 266) est positionné dans le bassin d'oxydation (43) en aval du premier tuyau d'alimentation (64; 264).

10. Système de bassin d'oxydation selon l'une quelconque des revendications 8 et 9, dans lequel chacun parmi les premier et second capteurs de qualité d'eau (92, 94) comprend en outre
au moins un élément de détection (104, 108) sélectionné dans le groupe constitué : des éléments de détection de sulfite (104, 401), et des éléments de détection de pH (108).

11. Système de bassin d'oxydation selon l'une quelconque des revendications 8 à 10, dans lequel les premier et second tuyaux d'alimentation (64, 66 ; 380, 382) sont pourvus de vannes de régulation individuelles (76, 78 ; 384 ; 386) pour commander la quantité de substance de renforcement d'oxydation introduite par le tuyau d'alimentation respectif (64, 66 ; 380, 382).

12. Système de bassin d'oxydation selon l'une quelconque des revendications 8 à 11, dans lequel les premier et second tuyaux d'alimentation (264, 266) sont raccordés à des dispositifs de soufflage individuels (276, 278) pour commander une quantité de gaz contenant de l'oxygène introduite à travers le tuyau d'alimentation respectif (264, 266).

13. Système de bassin d'oxydation selon l'une quelconque des revendications 8 à 12, dans lequel le système de bassin d'oxydation (42; 142) comprend 3 à 10 tuyaux d'alimentation consécutifs (64, 66, 68, 70, 72, 74; 264, 266, 268, 270, 272, 274), et 3 à 10 capteurs de qualité d'eau consécutifs (92, 94, 96, 98, 100, 102).

14. Système d'épuration d'un gaz de procédé à base d'eau de mer comprenant un laveur par voie humide (14) dans lequel un gaz de procédé est mis en contact avec de l'eau de mer pour éliminer le dioxyde de soufre à partir dudit gaz de procédé, et un système de bassin d'oxydation (42) selon l'une quelconque des revendications 8 à 13 pour traiter un effluent d'eau de mer (75) généré dans le laveur par voie humide (14) conjointement avec l'élimination du dioxyde de soufre à partir du gaz de procédé.
